# EUROPEAN PATENT APPLICATION

(11) **EP 3 264 747 A1**
(43) Date of publication of application: **03.01.2018**
(21) Application number: 16754894.0
(22) Date of filing: 26.01.2016
(51) Int. Cl.: H04N 5/64

(54) **DISPLAY DEVICE, DISPLAY METHOD AND PROGRAM**

(30) Priority: 24.02.2015 JP 2015033743
(71) Applicant: Seiko Epson Corporation, Tokyo 160-8801 (JP)
(72) Inventor: TANAKA, Hideki, Suwa-shi Nagano 392-8502 (JP); KITAZAWA, Takayuki, Suwa-shi Nagano 392-8502 (JP); MARUYAMA, Yuya, Suwa-shi Nagano 392-8502 (JP); OOUCHIDA, Yutaka, Sendai-shi Miyagi 980-8577 (JP); IZUMI, Shinichi, Sendai-shi Miyagi 980-8577 (JP)
(74) Representative: Hoffmann Eitle
(86) International application number: PCT/JP2016/000366
(87) International publication number: WO 2016/136137

(57) **Abstract**

A display device which enables training to be carried out efficiently, setting a condition suitable for the state of a patient as a condition for repetition of a movement, is provided.

A support device for phantom limb pain treatment 1 includes: a dynamic image transmitting unit 18 which outputs dynamic image data 13 in which a hand 3c carries out a repetition movement between a first state and a second state; a head-mounted display 2 which displays a dynamic image based on the dynamic image data 13; and a display condition input unit 16 which inputs a display condition for the repetition movement. The dynamic image transmitting unit 18 outputs the dynamic image data 13 corresponding to the display condition for the repetition movement. The display condition includes a forward speed which is a speed of shifting from the first state to the second state and a backward speed which is a speed of shifting from the second state to the first state.

## Description

### Technical Field

The present invention relates to a display device, a display method, and a program.

### Background Art

A patient who has a limb missing due to an accident or the like may have a sensation that there is a pain in the missing limb. This phenomenon is called phantom limb pain. With such a patient, a method in which the patient is made to think in such a way as to move the missing limb and is also shown an image of the missing limb moving, thus causing the patient to have the illusion that the limb is moving, has been found effective. That is, it is a method in which the patient is shown an image causing the patient to have the illusion that the missing limb actually exists. With this method, the recognition of the missing limb is properly carried out in the patient's brain and the pain disappears or is relieved.

Also, a device which causes a patient to see as if a paralyzed hand or missing hand were moving is disclosed in PTL 1. According to this, a plurality of magnetic sensors is installed on the patient' body. Then, a predetermined magnetic field is applied to the patient and the posture of the patient is detected. Then, a dynamic image of a hand is displayed on a display device. At this point, the position, posture and size of the hand in the dynamic image are adjusted so that the patient and the hand in the dynamic image are unified together.

The patient views the dynamic image and has the illusion that the hand in the dynamic image is a part of the patient' own body. For a patient with a missing hand, as the patient re-experiences the sense of unity with the hand in the brain, the pain of the hand disappears or is relieved. For a patient with a paralyzed hand, since a neural circuit is reconstructed in the brain, the paralysis of the hand is improved.

### Citation List

### Patent Literature

PTL 1: JP-A-2004-298430

### Summary of Invention

### Technical Problem

The dynamic image in PTL 1 is an image in which a predetermined movement is repeated and which is prepared in advance. In the case of the device of PTL 1, editing of a dynamic image is carried out by an editing device so as to suit to the patient, and training is carried out with a training device using the edited dynamic image. Therefore, it takes time and effort to prepare and edit the dynamic image so as to suit the patient. Thus, a display device which enables efficient training to be carried out using a dynamic image (video) suitable for the state of the patient has been desired.

### Solution to Problem

The invention has been made in order to solve the foregoing problem and can be realized in the following configurations or application examples.

### [Application Example 1]

A display device according to this application example includes: a dynamic image data output unit which outputs dynamic image data in which a target object carries out a repetition movement between a first state and a second state; a dynamic image display unit which displays a dynamic image based on the dynamic image data; and a display condition setting unit capable of setting a display condition for the repetition movement. The display condition includes a forward speed which is a speed of shifting from the first state to the second state and a backward speed which is a speed of shifting from the second state to the first state.

According to this application example, the display device has the dynamic image data output unit. The dynamic image data output unit outputs dynamic image data to the dynamic image display unit, and the dynamic image display unit displays a dynamic image based on the dynamic image data. In the dynamic image, the repetition movement between the first state and the second state is carried out. The patient views the dynamic image and sees the missing part of the body superimposed on the target object in the dynamic image. Then, the patient thinks in such a way that the missing part of the patient's own body carries out the repetition movement between the first state and the second state. This action serves as a treatment for reducing the pain of the missing part of the body.

In this treatment, when the forward speed and the backward speed are coincident with the patient's thought, the treatment can efficiently take place. Also, the display device according to this application example has the display condition setting unit, which inputs a forward speed and a backward speed. Also, since the dynamic image data is displayed under a display condition without being edited, the time taken for editing the dynamic image data is not necessary. Thus, a dynamic image in which the forward speed and the backward speed are changed each time to a speed suitable for the state of the patient can be displayed easily and therefore training can be carried out efficiently.

### [Application Example 2]

In the display device according to the foregoing application example, the dynamic image has a forward shift of shifting from the first state to the second state and a backward shift of shifting from the second state to the first state, a forward waiting state of waiting for a forward waiting time between the backward shift and the forward shift, and a backward waiting state of waiting for a backward waiting time between the forward shift and the backward shift. The display condition includes the forward waiting time and the backward waiting time.

According to this application example, the display device waits for the forward waiting time in the forward waiting state between the backward shift and the forward shift. Moreover, the display device waits for the backward waiting time in the backward waiting state between the forward shift and the backward shift. During the forward waiting time, the patient prepares for switching his/her thought from the backward shift to the forward shift. Similarly, during the backward waiting time, the patient prepares for switching his/her thought from the forward shift to the backward shift. Also, since the appropriate forward waiting time and backward waiting time change depending on the physical condition of the patient, it is preferable that an adjustment is made every time training is carried out. Moreover, in the display device according to this application example, the dynamic image can be adjusted, inputting the forward waiting time and the backward waiting time at the display condition setting unit. Therefore, a dynamic image where the forward waiting time and the backward waiting time are changed each time to a speed suitable for the state of the patient can be easily displayed and therefore training can be carried out efficiently.

### [Application Example 3]

In the display device according to the foregoing application example, the dynamic image includes images corresponding to a plurality of states of the target object. The display condition includes an image corresponding to the first state and an image corresponding to the second state.

According to this application example, the display condition for the repetition movement includes a screen corresponding to the first state and a screen corresponding to the second state. In the dynamic image, a transition is made across screens of a plurality of states of the target object. Also, since the appropriate screens of the first state and the second state change depending on the physical condition of the patient, it is preferable that an adjustment is made every time training is carried out. Moreover, in the display device according to this application example, the dynamic image can be adjusted, inputting the screens of the first state and the second state at the display condition setting unit. Therefore, a dynamic image where the screens of the first state and the second state are changed each time to a screen of a state suitable for the state of the patient can be easily displayed and therefore training can be carried out efficiently.

### [Application Example 4]

In the display device according to this application example, the dynamic image display unit is a head-mounted display.

According to this application example, the dynamic image display device is a head-mounted display and is used as it is installed on the patient's head. Therefore, the patient can move the place where the dynamic image is displayed, by moving his/her neck. Therefore, the dynamic image can be aligned with the missing target object according to the posture of the patient.

### [Application Example 5]

A display method according to this application example includes: displaying dynamic image data in which a repetition movement between a first state and a second state is carried out, according to a display condition for a dynamic image that is set. The display condition includes a forward speed which is a speed of shifting from the first state to the second state and a backward speed which is a speed of shifting from the second state to the first state.

According to this application example, the display condition for a dynamic image in which a repetition movement is carried out is inputted to a display condition setting unit. A storage unit stores dynamic image data. The dynamic image data includes data of a dynamic image in which a repetition movement between the first state and the second state is carried out. Then, the dynamic image data is inputted from the storage unit and a dynamic image corresponding to the display condition is displayed. The display condition includes the forward speed which is the speed of shifting from the first state to the second state and the backward speed which is the speed of shifting from the second state to the first state.

The patient, viewing the dynamic image, can carry out training so as to consciously move the missing part of the body with the dynamic image. Also, by inputting the forward speed and the backward speed to the display condition setting unit, the dynamic image can be displayed with a forward speed and a backward speed at which the dynamic image can be viewed easily. In this application example, since the dynamic image data is displayed under a display condition without being edited, the time taken for editing the dynamic image data is not necessary. Thus, a dynamic image in which the forward speed and the backward speed are changed each time to a speed suitable for the state of the patient can be displayed easily and therefore training can be carried out efficiently.

### [Application Example 6]

A program according to this application example causes a computer to function as: a display condition accepting unit which accepts a display condition for a dynamic image; and a dynamic image data output unit which outputs dynamic image data corresponding to the display condition to a display unit. The display condition includes a forward speed which is a speed at which a target object shifts from a first state to a second state and a backward speed which is a speed at which the target object shifts from the second state to the first state.

According to this application example, the program causes a computer to function as a display condition accepting unit which accepts a display condition for a dynamic image, and a dynamic image data output unit which outputs dynamic image data corresponding to the display condition to a display unit. The display condition includes the forward speed, which is the speed at which the target object shifts from the first state to the second state, and the backward speed, which is the speed at which the target object shifts from the second state to the first state.

The patient views the dynamic image and sees the missing part of the body superimposed on the body in the dynamic image. Then, the patient thinks in such a way that the missing part of the patient's own body carries out the repetition movement between the first state and the second state. This action serves as a treatment for reducing the pain of the missing part of the body. This treatment can be carried out efficiently when the forward speed and the backward speed are coincident with the patient's thought. Also, in this application example, the program causes the computer to function as a display condition accepting unit which accepts a display condition for the dynamic image. Therefore, the forward speed and the backward speed can be easily set and displayed each time to a speed suitable for the state of the patient and therefore training can be carried out efficiently.

### Brief Description of Drawings

[FIG. 1] FIG. 1 is a block diagram showing the configuration of a support device for phantom limb pain treatment according to a first embodiment.
[FIG. 2] FIG. 2 is a schematic view for explaining a display screen.
[FIG. 3] FIG. 3 is a flowchart of a phantom limb pain treatment method.
[FIG. 4] FIG. 4 is a schematic view for explaining the phantom limb pain treatment method.
[FIG. 5] FIG. 5 is a schematic view for explaining the phantom limb pain treatment method.
[FIG. 6] FIG. 6 is a view for explaining the phantom limb pain treatment method.
[FIG. 7] FIG. 7 is a view for explaining the phantom limb pain treatment method.
[FIG. 8] FIG. 8 is a schematic view showing the structure of a support device for phantom limb pain treatment according to a second embodiment.
[FIG. 9] FIG. 9 is a schematic view showing the structure of a support device for phantom limb pain treatment according to a third embodiment.
[FIG. 10] FIG. 10 is a schematic view for explaining a phantom limb pain treatment method according to a fourth embodiment.
[FIG. 11] FIG. 11 is a schematic view for explaining a phantom limb pain treatment method according to a fifth embodiment.

### Description of Embodiments

In the embodiments, characteristic examples of a support device for phantom limb pain treatment and a method for carrying out phantom limb pain treatment using this support device for phantom limb pain treatment will be described with reference to the drawings. Hereinafter, the embodiments will be described with reference to the drawings. Each member in each drawing is illustrated on a different scale from each other in order to achieve a recognizable size in each drawing.

### (First Embodiment)

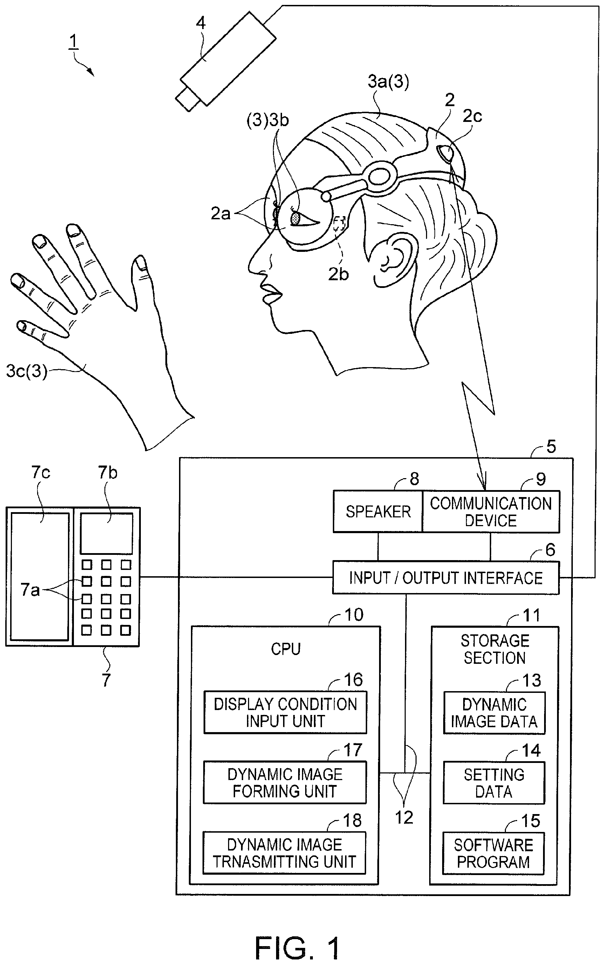

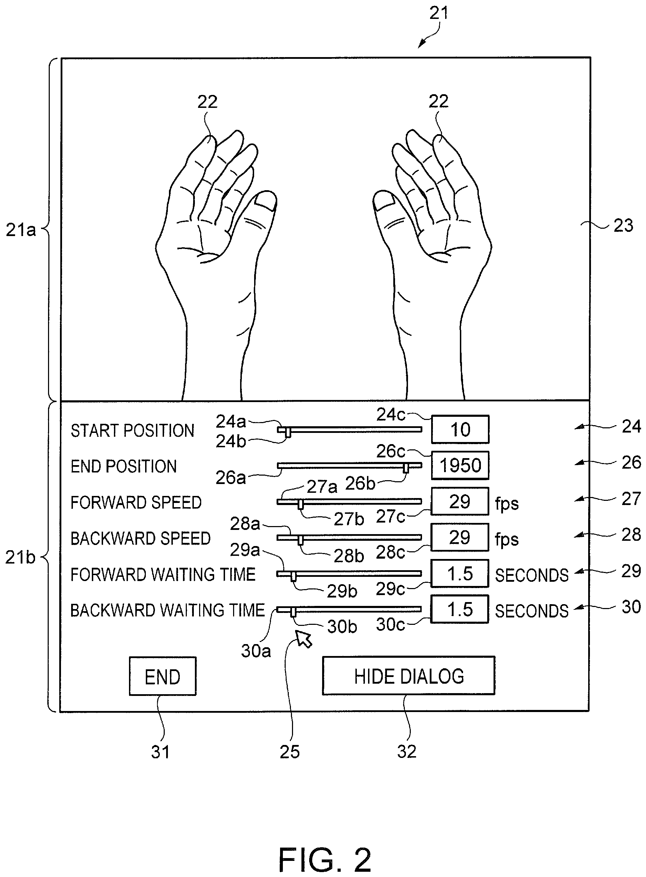

A support device for phantom limb pain treatment according to a first embodiment will be described with reference to FIG. 1 and FIG. 2. FIG. 1 is a block diagram showing the configuration of a support device for phantom limb pain treatment. As shown in FIG. 1, a support device for phantom limb pain treatment 1 has a head-mounted display 2 as a dynamic image display unit. The head-mounted display 2 is installed on a head 3a of a patient 3 (person). On the head-mounted display 2, a mirror part 2a is installed in positions facing eyes 3b of the patient 3. The head-mounted display 2 has a projection unit 2b. The projection unit 2b emits light to the mirror part 2a. The light is reflected by the mirror part 2a and becomes incident on the eyes 3b. The patient 3 can view a virtual dynamic image based on the light incident on the eyes 3b. The head-mounted display 2 can allow the right eye and the left eye to see different dynamic images from each other. Therefore, the head-mounted display 2 can allow the patient 3 to see a stereoscopic image in addition to a planar screen. The mirror part 2a is a non-transmitting mirror.

The support device for phantom limb pain treatment 1 has a camera 4. The camera 4 is capable of high-speed shooting and can shoot 300 screens per second. The camera 4 has an objective lens and a CCD (charge coupled device) image pickup element incorporated inside. The camera 4 has an objective lens with a long focusing range. The camera 4 takes in light reflected by an object present in a field of view, through the objective lens, and the light passing through the objective lens forms an image on the CCD image pickup element. Then, the image formed on the CCD image pickup element is converted to an electrical signal, thus enabling image pickup of the object present in the field of view. The camera 4 can use an image pickup tube or a CMOS (complementary metal-oxide semiconductor) image sensor instead of the CCD image pickup element. Moreover, an infrared image sensor may be used as well.

The head-mounted display 2 has a communication unit 2c. The support device for phantom limb pain treatment 1 has a control device 5, and the communication unit 2c communicates with and transmits and receives data to and from the control device 5. The communication unit 2c may employ wireless communication such as communication using radio waves as a medium or communication using light as a medium, or a configuration of wired communication. In this embodiment, for example, the communication unit 2c is a device which carries out Bluetooth communication.

The patient 3 has hands 3c as a part of the body. The patient 3 has one hand 3c missing, and the other hand 3c is in a healthy condition. The patient 3 carries out training to eliminate an itch or pain felt in the missing hand 3c, using support device for phantom limb pain treatment 1. In this embodiment, for example, it is assumed that the left hand of the patient 3 is healthy and the right hand is missing. The camera 4 is used when shooting an image of the left hand. The camera 4 shoots an image of the hand 3c and outputs the shot image to an input/output interface 6 of the control device 5.

The control device 5 has the input/output interface 6. An input/output terminal 7, a speaker 8, and a communication device 9 are connected to the input/output interface 6. The input/output terminal 7 has an input key 7a, a touch panel 7b, and a display unit 7c. The input key 7a is a button for the patient 3 to input an instruction content when operating the support device for phantom limb pain treatment 1. The touch panel 7b is a part to operate a point within an image displayed on the head-mounted display 2 and the display unit 7c. Touching the surface of the touch panel 7b with a finger and moving the finger thereon enables the pointer to be moved. Also, tapping the surface of the touch panel 7b enables an instruction to be given to select the site where the pointer is located. For the touch panel 7b, for example, an electrostatic capacitive sensor or pressure sensor can be used.

When the patient 3 wears the head-mounted display 2, it is difficult for the patient 3 to see the input key 7a. At this time, the patient 3 can feel around to operate the touch panel 7b so as to operate the pointer within the screen displayed on the head-mounted display 2 and thus can operate the support device for phantom limb pain treatment 1. On the display unit 7c, the same dynamic image or image as the dynamic image or image displayed on the head-mounted display 2 is displayed. An assistant who assists the patient 3 with the training can view the dynamic image on the display unit 7c and thus guide the patient 3. Moreover, the assistant can operate the input key 7a and the touch panel 7b and thus can operate the support device for phantom limb pain treatment 1.

The speaker 8 has the function of communicating a message to the patient 3 with an audio signal. When the patient 3 is undergoing a rehabilitation treatment, the control device 5 can communicate a message to the patient 3 from the speaker 8 even when the patient 3 is not concentrating his/her attention on the dynamic image projected on the mirror part 2a.

The communication device 9 is a device which communicates with the communication unit 2c installed in the head-mounted display 2. The communication device 9 and the communication unit 2c communicate data or the like of a dynamic image projected from the projection unit 2b.

In addition, the control device 5 has a CPU 10 (central processing unit) as a computing unit which carries out various kinds of computation processing as a processor, and a storage section 11 as a storage unit which stores various kinds of information. The input/output interface 6 and the storage section 11 are connected to the CPU 10 via a data bus 12.

The storage section 11 is a concept including a semiconductor memory such as RAM or ROM, a hard disk, or an external storage device such as DVD-ROM. Functionally, a storage area for storing dynamic image data 13 projected by the projection unit 2b is set. The dynamic image data 13 also includes data of an image shot by the camera 4. In addition, a storage area for storing setting data 14 of a playback condition or the like at the time of playing back the dynamic image of the dynamic image data 13 is set. In addition, a storage area for storing a software program 15 describing control procedures for the operation of the support device for phantom limb pain treatment 1 is set. In addition, a storage area which functions as a work area, temporarily file or the like for the CPU 10, and various other kinds of storage areas are set.

The CPU 10 is configured to control the support device for phantom limb pain treatment 1 according to the software program 15 stored in the storage section 11. The CPU 10 has a display condition input unit 16 which sets a condition for playing back a dynamic image, as a specific functional implementation unit. The display condition input unit 16 displays a screen which prompts an input of a playback condition for a dynamic image, on the head-mounted display 2 and the display unit 7c. Then, the patient 3 or the assistant operates the input key 7a or the touch panel 7b to input a display condition for playing back a dynamic image. Then, the display condition input unit 16 stores the playback condition for the dynamic image into the storage section 11 as the setting data 14.

In addition, the CPU 10 has a dynamic image forming unit 17. The dynamic image forming unit 17 shoots a dynamic image in which the healthy hand 3c moves, and stores the dynamic image into the storage section 11 as the dynamic image data 13. Moreover, the dynamic image forming unit 17 performs editing to add an inverted image of the dynamic image and stores the image into the storage section 11 as the dynamic image data 13.

In addition, the CPU 10 has a dynamic image transmitting unit 18 as a dynamic image data output unit. The dynamic image transmitting unit 18 has the function of transferring the data of the dynamic image data included in the dynamic image data 13 to the head-mounted display 2 and the display unit 7c. The dynamic image transmitting unit 18 has a memory which stores data corresponding to the display condition for playback, and the head-mounted display 2 has a memory which stores the data of the dynamic image. Then, the dynamic image transmitting unit 18 transfers the data of the dynamic image to the memory of the head-mounted display 2. In the head-mounted display 2, the projection unit 2b projects the dynamic image, using the dynamic image data transferred to the memory.

FIG. 2 is a schematic view for explaining a display screen. A display screen 21 shown in FIG. 2 is a screen displayed on the head-mounted display 2 and the display unit 7c. The display screen 21 has an image section 21a and a dialog section 21b. In the image section 21a, a dynamic image 23 in which an image 22 of a hand as a target object moves is displayed. In this embodiment, an example of training for the patient 3 having the hand 3c missing is employed, and in the dynamic image 23, a dynamic image shifting from the state where the hand 3c is open to the state where the hand 3c is closed and then shifting from the state where the hand 3c is closed to the state where the hand 3c is open is shown.

The state where the hand 3c is open is a first state. The state where the hand 3c is closed is a second state. A shift from the first state to the second state is a forward shift. A shift from the second state to the first state is a backward shift. Between the movement of the backward shift and the movement of the forward shift, a time period during which the movement of the image 22 of the hand is stopped is provided. The stopping of the movement of the image 22 of the hand at this time is referred to as forward waiting, and the waiting time is a forward waiting time. Similarly, between the movement of the forward shift and the movement of the backward shift, a time period during which the movement of the image 22 of the hand is stopped is provided. The stopping of the movement of the image 22 of the hand at this time is referred to as backward waiting, and the waiting time is a backward waiting time. Therefore, the movements of the image 22 of the hand in the dynamic image 23 are carried out in order of the forward shift, the backward waiting, the backward shift, and the forward waiting. Then, after the forward waiting, the movements are repeated again from the forward shift.

The dialog section 21b is a screen where the patient 3 or the assistant inputs and sets a display condition for playing back the dynamic image 23. A start position input section 24 is arranged at the top in the illustration of the dialog section 21b. In the start position input section 24, a frame which is an image used as the first state, of frames which are images included in the dynamic image, can be set. In the start position input section 24, a guide line 24a is arranged extending in the horizontal direction in the illustration, and a position indication mark 24b can move along the guide line 24a. A pointer 25 is displayed in the dialog section 21b. The pointer 25 is an arrow-shaped mark. The patient 3 and the assistant can move the pointer 25 by touching the touch panel 7b with a finger and moving the finger thereon.

The patient 3 and the assistant move the pointer 25 and superimpose the pointer 25 on the position indication mark 24b. Then, as the patient 3 and the assistant tap the touch panel 7b with a finger, an input to the start position input section 24 is enabled. Then, the position indication mark 24b can be moved to the left and right, linked with the movement of the pointer 25. When the pointer 25 moves to the left and right, the image displayed in the image section 21a is switched, corresponding to the position of the position indication mark 24b.

When the position indication mark 24b is moved to the left end of the guide line 24a, the first image of the dynamic image that is shot is displayed in the image section 21a. When the position indication mark 24b is moved to the right end of the guide line 24a, the last image of the dynamic image that is shot is displayed in the image section 21a. Then, when the patient 3 and the assistant move the position of the position indication mark 24b, the image displayed in the image section 21a is switched. The patient 3 and the assistant view the screen displayed in the image section 21a and select the first state. After selecting the first state, the patient 3 and the assistant taps the touch panel 7b with a finger. Thus, the position indication mark 24b stops moving and the image of the first state is decided.

A start position space 24c is arranged on the right-hand side of the guide line 24a in the illustration. In the start position space 24c, the frame number of the screen indicated by the position indication mark 24b is displayed. When the position indication mark 24b is located at the left end of the guide line 24a, the numeral 1 is displayed in the start position space 24c. When the position indication mark 24b is located at the right end of the guide line 24a, a numeral corresponding to the last frame number of the dynamic image that is shot is displayed in the start position space 24c. Then, when the position indication mark 24b is moved, a number corresponding to the position of the position indication mark 24b is displayed in the start position space 24c.

An end position input section 26 is arranged below the start position input section 24 in the illustration. In the end position input section 26, a frame which is an image used as the second state, of frames which are images included in the dynamic image, can be set. In the end position input section 26, a guide line 26a is arranged extending in the horizontal direction in the illustration, and a position indication mark 26b can move along the guide line 26a. The patient 3 and the assistant can move the position indication mark 26b, using the pointer 25.

Similarly to the operation of the start position input section 24, when the patient 3 and the assistant move the position of the position indication mark 26b, the image displayed in the image section 21a is switched. The patient 3 and the assistant views the screen displayed in the image section 21a and selects a frame to be used as the second state. After selecting the second state, the patient 3 and the assistant taps the touch panel 7b with a finger. Thus, the position indication mark 26b stops moving and the second state is decided. An end position space 26c is arranged on the right-hand side of the guide line 26a in the illustration. In the end position space 26c, the frame number of the screen indicated by the position indication mark 26b is displayed. Then, when the position indication mark 26b is moved, a number corresponding to the position of the position indication mark 26b is displayed in the end position space 26c.

A forward speed input section 27 as a display condition setting unit is arranged below the end position input section 26 in the illustration. In the forward speed input section 27, the playback speed of the dynamic image 23 in the forward shift can be set. In the forward speed input section 27, a guide line 27a is arranged extending in the horizontal direction in the illustration, and a speed indication mark 27b can move along the guide line 27a. The patient 3 and the assistant can move the speed indication mark 27b, using the pointer 25.

A forward speed space 27c is arranged on the right-hand side of the guide line 27a in the illustration. In the forward speed space 27c, the playback speed of the dynamic image 23 indicated by the speed indication mark 27b is displayed. Then, when the speed indication mark 27b is moved, a speed corresponding to the position of the speed indication mark 27b is displayed in the forward speed space 27c. The unit for the playback speed of the dynamic image 23 is fps (frames per second). In this embodiment, for example, the recording speed of the dynamic image 23 shot by the camera 4 is 300 fps. Then, the playback speed that can be designated in the forward speed input section 27 is 1 to 300 fps. When the playback speed is 10 fps, it means that the dynamic image is played back at a speed one-tenth of the recording speed. Therefore, the support device for phantom limb pain treatment 1 can play back the movement of the hand 3c at a low speed in the forward shift. After selecting the playback speed in the forward shift, the patient 3 and the assistant tap the touch panel 7b with a finger. Thus, the speed indication mark 27b stops moving and the playback speed in the forward shift is decided.

A backward speed input section 28 as a display condition setting unit is arranged below the forward speed input section 27 in the illustration. In the backward speed input section 28, the playback speed of the dynamic image 23 in the backward shift can be set. In the backward speed input section 28, a guide line 28a is arranged extending in the horizontal direction in the illustration, and a speed indication mark 28b can move along the guide line 28a. The patient 3 and the assistant can move the speed indication mark 28b, using the pointer 25.

A backward speed space 28c is arranged on the right-hand side of the guide line 28a in the illustration. In the backward speed space 28c, the playback speed of the dynamic image 23 indicated by the speed indication mark 28b is displayed. Then, when the speed indication mark 28b is moved, a speed corresponding to the position of the speed indication mark 28b is displayed in the backward speed space 28c. The playback speed that can be designated in the backward speed input section 28 is 1 to 300 fps. Therefore, the support device for phantom limb pain treatment 1 can play back the movement of the hand 3c at a low speed in the backward shift. After selecting the playback speed in the backward shift, the patient 3 and the assistant tap the touch panel 7b with a finger. Thus, the speed indication mark 28b stops moving and the playback speed in the backward shift is decided.

A forward waiting time input section 29 is arranged below the backward speed input section 28 in the illustration. In the forward waiting time input section 29, the time of forward waiting can be set. In the forward waiting time input section 29, a guide line 29a is arranged extending in the horizontal direction in the illustration, and a time indication mark 29b can move along the guide line 29a. The patient 3 and the assistant can move the time indication mark 29b, using the pointer 25.

A forward waiting time space 29c is arranged on the right-hand side of the guide line 29a in the illustration. In the forward waiting time space 29c, the time indicated by the time indication mark 29b is displayed. Then, when the time indication mark 29b is moved, a time corresponding to the position of the time indication mark 29b is displayed. Also, the time that can be designated in the forward waiting time input section 29 is 0.1 to 10 seconds. Therefore, the support device for phantom limb pain treatment 1 can prepare sufficiently during a preparation time for changing the movement of the hand 3c from the backward shift to the forward shift. After selecting the forward waiting time, the patient 3 and the assistant tap the touch panel 7b with a finger. Thus, the time indication mark 29b stops moving and the time of forward waiting is decided.

A backward waiting time input section 30 is arranged below the forward waiting time input section 29 in the illustration. In the backward waiting time input section 30, the time of backward waiting can be set. In the backward waiting time input section 30, a guide line 30a is arranged extending in the horizontal direction in the illustration, and a time indication mark 30b can move along the guide line 30a. The patient 3 and the assistant can move the time indication mark 30b, using the pointer 25.

A backward waiting time space 30c is arranged on the right-hand side of the guide line 30a in the illustration. In the backward waiting time space 30c, the time indicated by the time indication mark 30b is displayed. Then, when the time indication mark 30b is moved, a time corresponding to the position of the time indication mark 30b is displayed. Also, the time that can be designated in the backward waiting time input section 30 is 0.1 to 10 seconds. Therefore, the support device for phantom limb pain treatment 1 can prepare sufficiently during a preparation time for changing the movement of the hand 3c from the forward shift to the backward shift. After selecting the backward waiting time, the patient 3 and the assistant tap the touch panel 7b with a finger. Thus, the time indication mark 30b stops moving and the time of backward waiting is decided.

An end mark 31 and a hide-dialog mark 32 are arranged below the backward waiting time input section 30 in the illustration. When the patient 3 and the assistant place the pointer 25 on the end mark 31 and tap the touch panel 7b with a finger, the operation of the support device for phantom limb pain treatment 1 can be stopped. When the patient 3 and the assistant place the pointer on the hide-dialog mark 32 and tap the touch panel 7b with a finger, the display of the dialog section 21b can be stopped and the area of the image section 21a can be increased. The startup of the support device for phantom limb pain treatment 1 and the redisplay of the dialog section 21b can be carried out by operating the input key 7a.

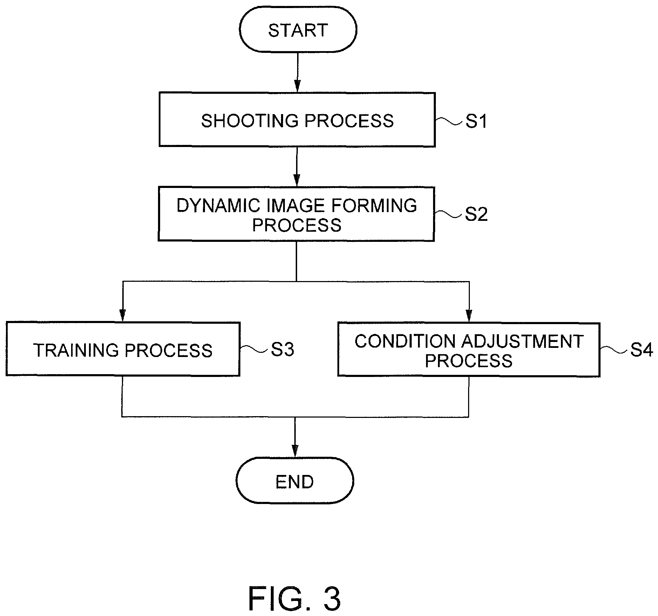

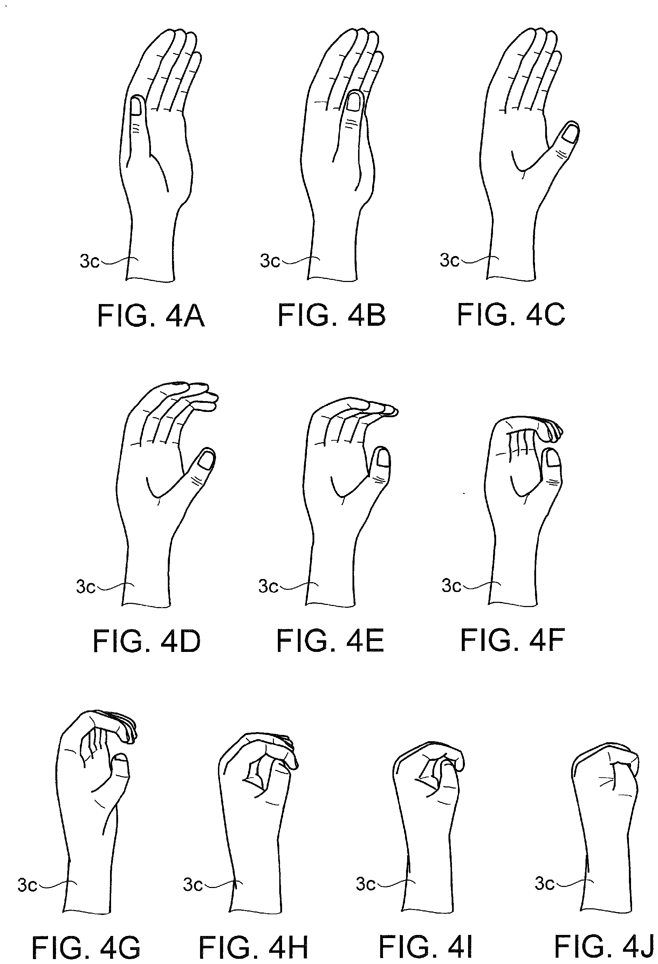

Next, a phantom limb pain treatment method using the support device for phantom limb pain treatment 1 described above will be described with reference to FIGS. 3 to 7. FIG. 3 is a flowchart of the phantom limb pain treatment method. FIGS. 4 to 7 are views for explaining the phantom limb pain treatment method. In the flowchart of FIG. 3, Step S1 is equivalent to a shooting process. It is the process of shooting the healthy hand 3c of the patient 3 with its shape changed from an open state to a closed state. Next, the processing shifts to Step S2. Step S2 is a dynamic image forming process. This process is the process of editing a shot dynamic image 23 and forming a dynamic image 23 for training. Next, the processing shifts to Step S3 and Step S4. Step S3 and Step S4 are carried out in parallel.

Step S3 is a training process. This process is the process in which the patient 3 views the dynamic image and thinks to move the missing hand 3c. Step S4 is a condition adjustment process. This process is the process of changing the range of repeatedly playing back the dynamic image 23, the playback speed, the forward waiting time, and the backward waiting time. When Step S3 ends, the phantom limb pain treatment ends.

Next, the phantom limb pain treatment method will be described in detail, using FIGS. 4 to 7 and corresponding to the steps shown in FIG. 3. FIG. 4 is a view corresponding to the shooting process of Step S1. As shown in FIG. 4, the hand 3c of the patient 3 is shot, with its shape gradually changed from an open state to a closed state. In this embodiment, since the patient 3 has his/her right hand missing, the left hand is shot. A shot image of the hand 3c shown in FIG. 4(a) is a first image. A shot image of the hand 3c shown in FIG. 4(b) is a second image. Similarly, a shot image of the hand 3c shown in FIG. 4(c) is a third image. A shot image of the hand 3c shown in FIG. 4 (d) is a fourth image. A shot image of the hand 3c shown in FIG. 4(e) is a fifth image. A shot image of the hand 3c shown in FIG. 4(f) is a sixth image. A shot image of the hand 3c shown in FIG. 4 (g) is a seventh image. A shot image of the hand 3c shown in FIG. 4(h) is an eighth image. A shot image of the hand 3c shown in FIG. 4(i) is a ninth image. A shot image of the hand 3c shown in FIG. 4(j) is a tenth image.

The first to tenth images are sequential images. Also, each image is shot at the shooting speed of 300 fps and therefore shot at a higher speed than with an ordinary camera. Therefore, if the playback speed is reduced to below 300 fps, the images can be played back at a lower speed than the speed at which the images are shot.

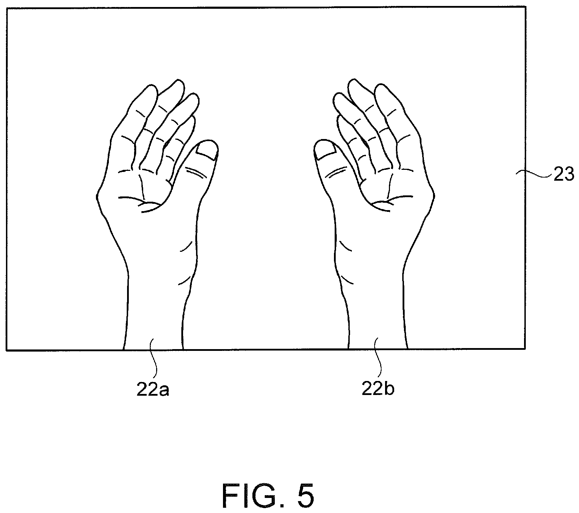

FIG. 5 is a view corresponding to the dynamic image forming process of Step S2. As shown in FIG. 5, in Step S2, an image 22b of the right hand, which is a duplicate of an image 22a of the left hand inverted in the left-right direction, is formed. Then, the image 22a of the left hand and the image 22b of the right hand are juxtaposed to the left and right. Then, based on these images, a sequential forward dynamic image 23a continuing from the first image to the tenth image is formed. The forward dynamic image 23a is a dynamic image 23 changing from the image 22 of the hand in which the hand 3c is open to the image 22 of the hand in which the hand 3c is closed. Moreover, a backward dynamic image 23b, which is the forward dynamic image 23a played back in reverse, is formed. The backward dynamic image 23b is a dynamic image 23 changing from the image 22 of the hand in which the hand 3c is closed to the image 22 of the hand in which the hand 3c is open.

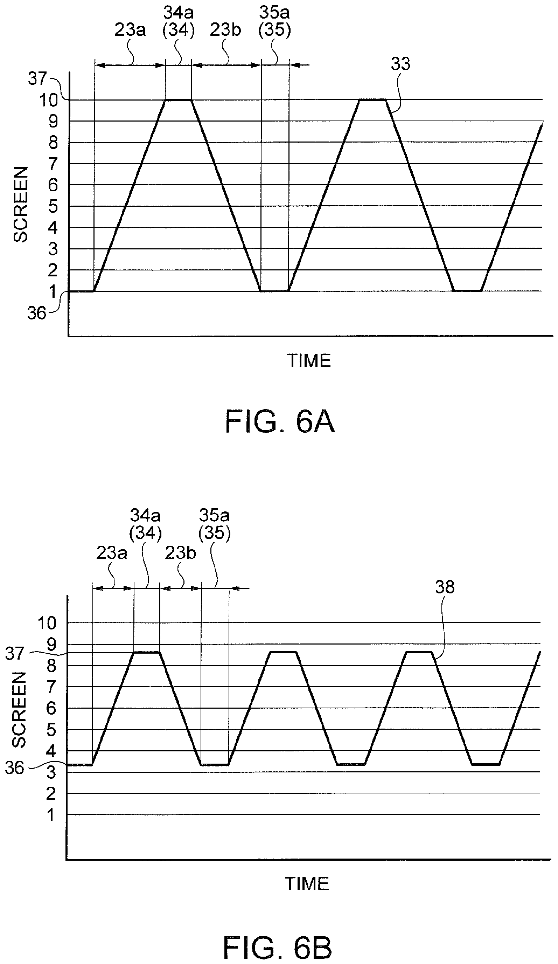

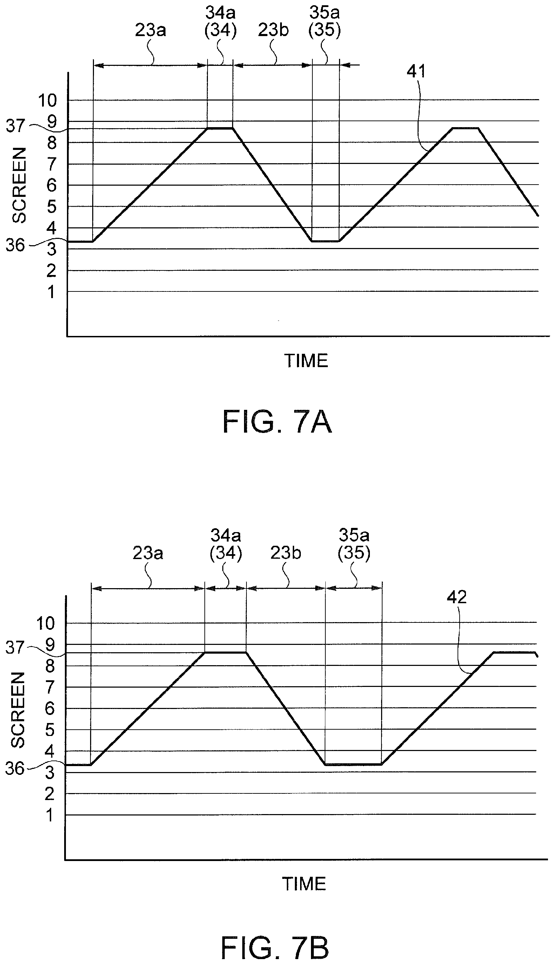

FIG. 6 and FIG. 7 are views corresponding to the training process of Step S3 and the condition adjustment process of Step S4. FIG. 6 (a) to FIG. 7 (b) are time charts showing the progress of the dynamic image 23. The vertical axis represents screen numbers projected in the dynamic image 23. The horizontal axis represents the transition of time. Time shifts from the left-hand side to the right-hand side in the illustrations. In Step 3, the support device for phantom limb pain treatment 1 plays back the dynamic image 23. Then, the patient 3 views the dynamic image 23 and thinks to open and close the hand 3c synchronously with the image 22 of the hand. The patient 3 has the right hand 3c missing and therefore cannot physically open and close the right hand 3c but imagines and thinks to open and close the hand 3c in the brain. It is preferable that the left hand 3c is opened and closed with the dynamic image 23. Thus, the patient tends to have a sensation of the right hand 3c opening and closing synchronously.

A first transition line 33 in FIG. 6(a) indicates the progress of the dynamic image 23. As indicated by the first transition line 33, the forward dynamic image 23a from the first image to the tenth image is played back first. At this time, the patient 3 thinks to turn the hand 3c from the open state to the closed state while viewing the dynamic image 23. After the support device for phantom limb pain treatment 1 plays back the forward dynamic image 23a, backward waiting 34 as a backward waiting state is carried out. In the backward waiting 34, the dynamic image 23 stops for a backward waiting time 34a. During this time, the patient 3 prepares for the backward shift. After the backward waiting 34, the support device for phantom limb pain treatment 1 plays back the backward dynamic image 23b from the tenth image to the first image. At this time, the patient 3 thinks to turn the hand 3c from the closed state to the open state. Then, after the support device for phantom limb pain treatment 1 plays back the backward dynamic image 23b, forward waiting 35 as a forward waiting state is carried out. In the forward waiting 35, the dynamic image 23 stops for a forward waiting time 35a. During this time, the patient 3 prepares for the forward shift. Repeating the above contents, the training is carried out.

The state of the hand 3c shown on the screen at the time of starting the forward dynamic image 23a is a first state 36. Then, the state of the hand 3c shown on the screen at the time of ending the forward dynamic image 23a is a second state 37. In Step S3, the patient 3 carries out a repetition movement of moving the hand 3c between the first state 36 and the second state 37. Then, on the first transition line 33, the first state 36 is the state shown by the first image, and the second state 37 is the image shown by the tenth image.

First, the patient 3 places the hand 3c in the first state 36. The first state 36 is the state where the patient 3 has the hand 3c open. Next, the patient 3 turns the hand 3c into the second state 37 while viewing the forward dynamic image 23a. That is, the patient 3 turns the hand 3c from the open state to the closed state. Next, the patient 3 carries out the backward waiting 34 during the backward waiting time 34a. During this time, the patient 3 maintains the hand 3c in the closed state. Then, the patient 3 prepares to open the hand 3c.

In the backward waiting 34, the hand 3c is in the second state 37. The second state 37 is the state where the patient 3 has the hand 3c closed. Next, the patient 3 turns the hand 3c into the first state 36 while viewing the backward dynamic image 23b. That is, the patient 3 turns the hand 3c from the closed state to the open state. Next, the patient 3 carries out the forward waiting 35 during the forward waiting time 35a. During this time, the patient 3 maintains the hand 3c in the open state. Then, the patient 3 prepares to close the hand 3c. As indicated by the first transition line 33, subsequently the training while viewing the forward dynamic image 23a and the training while viewing the backward dynamic image 23b are carried out repeatedly.

The support device for phantom limb pain treatment 1 can carry out the condition adjustment process of Step S4, interrupting the training process of Step S3. Next, an example in which the first state 36 and the second state 37 of the dynamic image 23 are changed will be described. The patient 3 or the assistant moves the position indication mark 24b indicating the start position, from the position indicating the first image to a position indicating an image between the third image and the fourth image. Moreover, the patient 3 or the assistant moves the position indication mark 26b indicating the end position, from the position indicating the tenth image to a position indicating an image between the eighth image and the ninth image. Then, the training process of Step S3 is resumed. In Step S3, the dynamic image transmitting unit 18 outputs the dynamic image data in which the first state 36 and the second state 37 are changed, to the head-mounted display 2. Then, the head-mounted display 2 displays the dynamic image 23 in which the first state 36 and the second state 37 are changed. The dynamic image transmitting unit 18 only changes the playback condition and causes the dynamic image data 13 to be played back without making any change thereto.

A second transition line 38 in FIG. 6(b) indicates the progress of the dynamic image 23. As indicated by the second transition line 38, the forward dynamic image 23a is started from the image between the third image and the fourth image and is played back up to the image between the eighth image and the ninth image. At this time, the patient 3 thinks to turn the hand 3c from a slightly open state to a slightly closed state. After the playback of the forward dynamic image 23a, the backward waiting 34 is carried out and then the backward dynamic image 23b is played back. The backward dynamic image 23b is started from the image between the eighth image and the ninth image and is played back up to the image between the third image and the fourth image. At this time, the patient 3 thinks to turn the hand 3c from the slightly closed state to the slightly open state. After the playback of the backward dynamic image 23b, the forward waiting 35 is carried out and then the forward dynamic image 23a is played back. Repeating the above contents, the training is carried out.

When the patient is not accustomed to the training of thinking of the opening/closing of the hand 3c in the first state 36, thinking may be easier if the opening/closing of the hand 3c is narrower. In this case, the training is carried out, adjusting the first state 36 and the second state 37 and thus setting the dynamic image 23 showing a state which is easier for the patient 3 to think of. Meanwhile, when the patient has become accustomed to the training, the training is carried out, adjusting the first state 36 and the second state 37 and thus setting the dynamic image 23 showing a state which is easier for the patient 3 to think of. Thus, the training can be carried out efficiently.

Next, an example in which the forward speed, which is the speed of playing back the forward dynamic image 23a of the dynamic image 23, and the backward speed, which is the speed of playing back the backward dynamic image 23b, are changed will be described. The patient 3 or the assistant moves the speed indication mark 27b in the forward speed input section 27 indicating the forward speed. The speed indication mark 27b is moved to reduce the numeral in the forward speed space 27c. Moreover, the patient 3 or the assistant moves the speed indication mark 28b in the backward speed input section 28 indicating the backward speed. The speed indication mark 28b is moved to reduce the numeral in the backward speed space 28c. Then, the training process of Step S3 is resumed. In Step S3, the dynamic image transmitting unit 18 outputs the dynamic image data in which the forward speed and the backward speed are changed, to the head-mounted display 2. Then, the head-mounted display 2 displays the dynamic image 23 in which the forward speed and the backward speed are changed. The dynamic image transmitting unit 18 only changes the playback condition and causes the dynamic image data 13 to be played back without making any change thereto.

A third transition line 41 in FIG. 7(a) indicates the progress of the dynamic image 23. As indicated by the third transition line 41, the playback time of the forward dynamic image 23a is longer than the playback time of the forward dynamic image 23a indicated by the second transition line 38. Therefore, on the third transition line 41, the forward speed is slower. Similarly, as indicated by the third transition line 41, the playback time of the backward dynamic image 23b is longer than the playback time of the backward dynamic image 23b indicated by the second transition line 38. Therefore, on the third transition line 41, the backward speed is slower.

When the patient is not accustomed to the training of thinking of the opening/closing of the hand 3c, thinking may be difficult if the speed of opening and closing the hand 3c is fast. In this case, the training is carried out, slowing down the forward speed and the backward speed and thus setting the dynamic image 23 of a state which is easier for the patient 3 to think of. Meanwhile, when the patient has become accustomed to the training, the training is carried out, increasing the forward speed and the backward speed and thus setting the dynamic image 23 of a state which is easier for the patient 3 to think of. Thus, the training can be carried out efficiently.

Next, an example in which the forward waiting time 35a and the backward waiting time 34a of the dynamic image 23 are changed will be described. The patient 3 or the assistant moves the time indication mark 29b in the forward waiting time input section 29 indicating the forward waiting time 35a. The time indication mark 29b is moved to increase the numeral in the forward waiting time space 29c. Moreover, the patient 3 or the assistant moves the time indication mark 30b in the backward waiting time input section 30 indicating the backward waiting time 34a. The time indication mark 30b is moved to increase the numeral in the backward waiting time space 30c. Then, the training process of Step S3 is resumed. In Step S3, the dynamic image transmitting unit 18 outputs the dynamic image data in which the forward waiting time 35a and the backward waiting time 34a are changed, to the head-mounted display 2. Then, the head-mounted display 2 displays the dynamic image 23 in which the forward waiting time 35a and the backward waiting time 34a are changed. The dynamic image transmitting unit 18 only changes the playback condition and causes the dynamic image data 13 to be played back without making any change thereto.

A fourth transition line 42 in FIG. 7(b) indicates the progress of the dynamic image 23. As indicated by the fourth transition line 42, the backward waiting time 34a is longer than the backward waiting time 34a indicated by the third transition line 41. Therefore, on the fourth transition line 42, the time until the backward dynamic image 23b is started after the forward dynamic image 23a ends is longer. Similarly, as indicated by the fourth transition line 42, the forward waiting time 35a is longer than the forward waiting time 35a indicated by the third transition line 41. Therefore, on the fourth transition line 42, the time until the forward dynamic image 23a is started after the backward dynamic image 23b ends is longer.

When the patient is not accustomed to the training of thinking of the opening/closing of the hand 3c, thinking may be difficult if the forward waiting time 35a and the backward waiting time 34a are short. In this case, the forward waiting time 35a and the backward waiting time 34a are made longer. Then, the training is carried out, setting the state where it is easier for the patient 3 to prepare to think, during the forward waiting time. Meanwhile, when the patient has become accustomed to the training, the training is carried out, reducing the forward waiting time 35a and the backward waiting time 34a and thus setting the dynamic image 23 of a state which is easier for the patient 3 to think of. Thus, the training can be carried out efficiently.

As described above, this embodiment has the following effects.
(1) According to this embodiment, the dynamic image transmitting unit 18 outputs dynamic image data to the head-mounted display 2, and the head-mounted display 2 displays the dynamic image 23 based on the dynamic image data. In the dynamic image 23, the image of the hand 3c in the first state 36 to the image of the hand 3c in the second state 37 are repeatedly shown. The patient 3 views the dynamic image 23 and sees the hand 3c superimposed on the image 22 of the hand in the dynamic image 23. Then the patient 3 thinks to carry out a repetition movement between the first state 36 and the second state 37. By this training, the pain in the missing hand 3c can be relieved.
(2) According to this embodiment, when the forward speed and the backward speed in the training are coincident with the speeds which the patient thinks of, the treatment can be carried out efficiently. Also, the support device for phantom limb pain treatment 1 has the forward speed input section 27 which input the forward speed and the backward speed input section 28 which inputs the backward speed. Since the dynamic image data 13 is displayed under a display condition without being edited, the time taken for editing the dynamic image data 13 is not necessary. Therefore, the dynamic image in which the forward speed and the backward speed are changed each time to a speed suitable for the state of the patient can be displayed and therefore the training can be carried out efficiently.
(3) According to this embodiment, the support device for phantom limb pain treatment 1 waits for the forward waiting time 35a in the forward waiting 35. Moreover, the support device for phantom limb pain treatment 1 waits for the backward waiting time 34a in the backward waiting 34. During the forward waiting time 35a, the patient 3 prepares to switch his/her thought from the backward shift to the forward shift. Similarly, during the backward waiting time 34a, the patient 3 prepares to switch his/her thought from the forward shift to the backward shift. Then, since the forward waiting time 35a and the backward waiting time 34a change depending on the physical condition of the patient 3, it is preferable that an adjustment is made every time training is carried out. Also, the support device for phantom limb pain treatment 1 in this embodiment can input the forward waiting time 35a and the backward waiting time 34a via the display condition input unit 16 and thus adjust the dynamic image. Therefore, the dynamic image in which the forward waiting time 35a and the backward waiting time 34a are changed each time to a speed suitable for the state of the patient 3 can be displayed and therefore training can be carried out efficiently.
(4) According to this embodiment, the display condition for the repetition movement includes an image corresponding to the first state 36 and an image corresponding to the second state 37, of the dynamic image 23. In the dynamic image 23, a transition is made across images of a plurality of states. Also, since the appropriate first state 36 and second state 37 change depending on the physical condition of the patient 3, it is preferable that an adjustment is made every time training is carried out. Moreover, the support device for phantom limb pain treatment 1 can input the first state 36 and the second state 37 via the display condition setting unit 16. Therefore, the screens of the first state 36 and the second state 37 can be changed each time to the dynamic image 23 suitable for the state of the patient 3 and thus displayed and therefore the training can be carried out efficiently.
(5) According to this embodiment, the site where the dynamic image 23 is displayed is the head-mounted display 2, which is used as it is installed on the head 3a of the patient 3. Therefore, the patient 3 can move the place where the dynamic image 23 is displayed, by moving his/her neck. Therefore, the dynamic image 23 can be aligned with the missing part of the body according to the posture of the patient 3.

### (Second Embodiment)

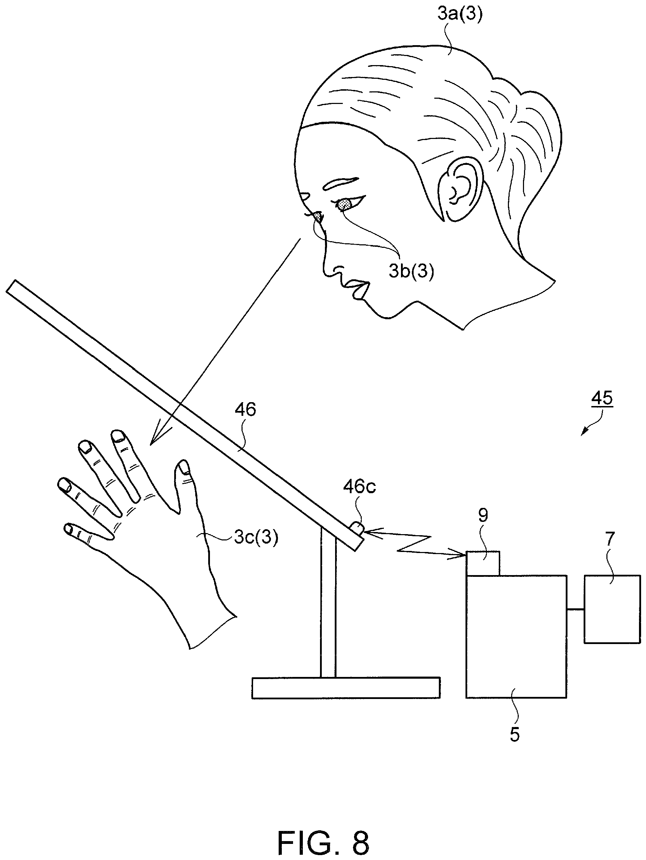

Next, an embodiment of the support device for phantom limb pain treatment will be described, using the schematic plan view of FIG. 8 showing a structure of the support device for phantom limb pain treatment. The difference of this embodiment from the first embodiment is that the dynamic image 23 is displayed on a display panel. The explanation of the same features as in the first embodiment is omitted.

That is, in this embodiment, a support device for phantom limb pain treatment 45 has a display device 46, as shown in FIG. 8. The display device 46 is a flat panel display or a display with a curved surface. As the display device 46, a liquid crystal display or an OLED (organic light-emitting diodes) display can be used. A communication unit 46c is installed in the display device 46. The communication unit 46c can receive data of the dynamic image 23 from the communication device 9.

The display device 46 is arranged between the head 3a and the hand 3c of the patient 3. Then, the patient 3 can carry out the training of thinking to open and close the missing hand 3c while viewing the dynamic image 23 displayed on the display device 46. In this case, too, the patient 3 or the assistant can see the dynamic image 23, inputting a display condition for the dynamic image 23 with the use of the input/output terminal 7 and thus easily changing the playback condition each time.

### (Third Embodiment)

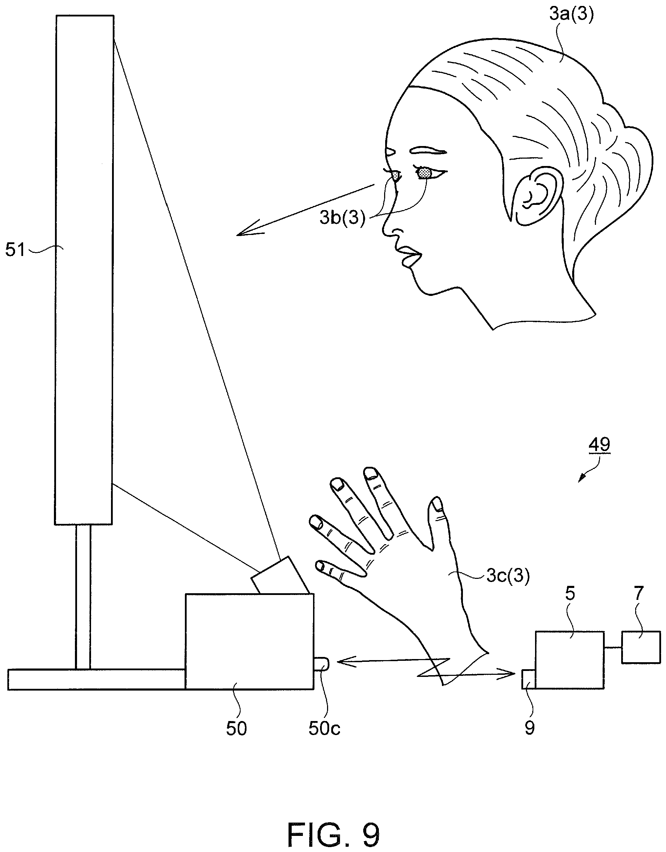

Next, an embodiment of the support device for phantom limb pain treatment will be described, using the schematic plan view of FIG. 9 showing a structure of the support device for phantom limb pain treatment. The difference of this embodiment from the first embodiment is that the dynamic image 23 is displayed on a screen, using a projector. The explanation of the same features as in the first embodiment is omitted.

That is, in this embodiment, a support device for phantom limb pain treatment 49 has a projector 50 and a screen 51, as shown in FIG. 9. A communication unit 50c is installed in the projector 50. The communication unit 50c can receive data of the dynamic image 23 from the communication device 9.

The screen 51 is installed in front of the patient 3. Then, the patient 3 can carry out the training of thinking to open and close the missing hand 3c while viewing the dynamic image 23 displayed on the screen 51. In this case, too, the patient 3 or the assistant can see the dynamic image 23, inputting a display condition for the dynamic image 23 with the use of the input/output terminal 7 and thus easily changing the playback condition each time.

### (Fourth Embodiment)

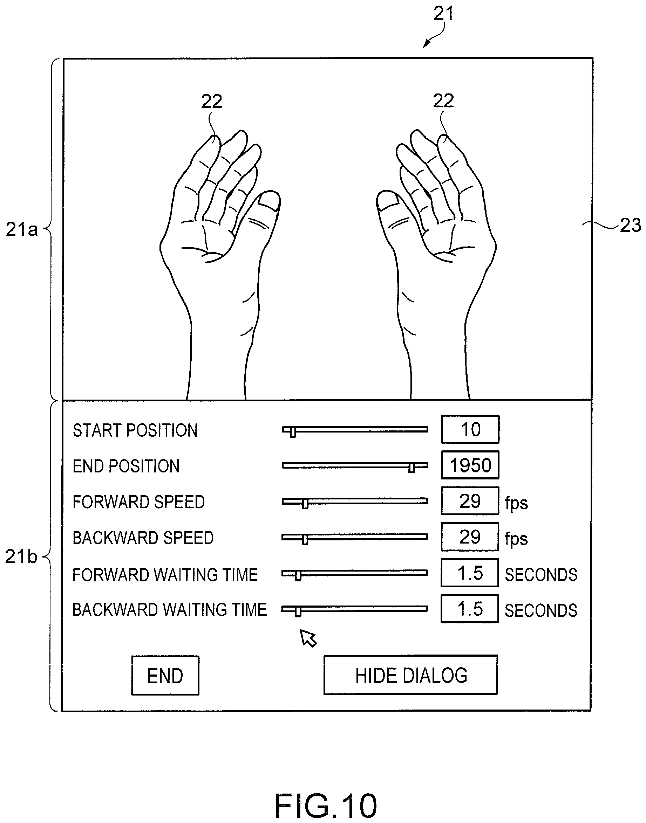

Next, an embodiment of the support device for phantom limb pain treatment will be described, using the schematic view of FIG. 10 for explaining a phantom limb pain treatment method. The difference of this embodiment from the first embodiment is that the dynamic image data 13 is not of a shot image of the hand 3c of the patient 3 but is the dynamic image data 13 of a shot image of a hand 3c of another person.

That is, in this embodiment, the dynamic image 23 is displayed in the image section 21a, as shown in FIG. 10. In this dynamic image 23, the image 22 of a hand of an able-bodied person is shown. In the shooting process of Step S1, shooting is carried out in the state where the able-bodied person is opening and closing the hand 3c. Then, in the training process of Step S3, the patient 3 carries out training, observing the dynamic image 23 in which the able-bodied person is opening and closing the hand 3c. Therefore, even when the patient 3 has both the left and right hands 3c missing, the patient 3 can carry out the training using the support device for phantom limb pain treatment 1. Then, in this case, too, the assistant can input a display condition for the dynamic image 23, using the input/output terminal 7, and the patient 3 can view the dynamic image 23, changing the playback condition each time.

### (Fifth Embodiment)

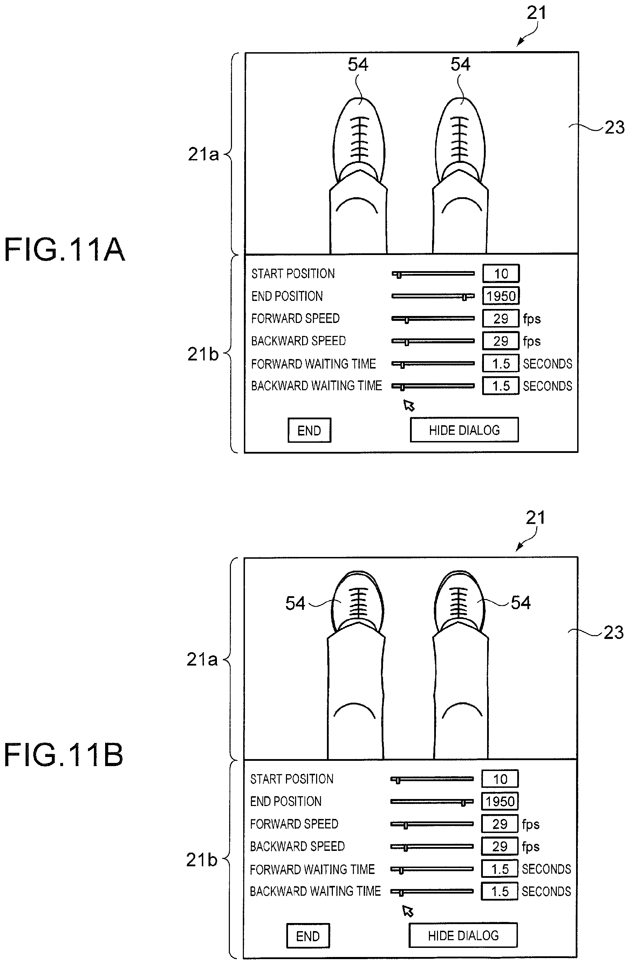

Next, an embodiment of the support device for phantom limb pain treatment will be described, using the schematic view of FIG. 11 for explaining a phantom limb pain treatment method. The difference of this embodiment from the first embodiment is that the dynamic image data 13 is not a shot image of the hand 3c but is the dynamic image data 13 of a shot image of a foot.

That is, in this embodiment, the dynamic image 23 is displayed in the image section 21a, as shown in FIG. 11. In this dynamic image 23, an image 54 of a foot is shown. On the screen of the dynamic image 23 in FIG. 11(a), the state where a person is sitting on a chair, with his/her knees bent and his/her feet dropped, is shown. On the screen of the dynamic image 23 in FIG. 11(b), the state where a person is sitting on a chair, with his/her knees extended and his/her feet lifted up, is shown.

In the shooting process of Step S1, a movement in which the patient 3 sitting on a chair moves his/her foot up and down is shot. In the dynamic image forming process of Step S2, a dynamic image of a left-right symmetric mirror image is formed. Then, in the training process of Step S3, the patient 3 carries out training, observing the dynamic image 23 in which the feet are moved up and down. Therefore, even when the patient 3 has one foot missing, the patient 3 can carry out the training using the support device for phantom limb pain treatment 1. Then, in this case, too, the patient 3 or the assistant can input a display condition for the dynamic image 23, using the input/output terminal 7, and the patient 3 can view the dynamic image 23, easily changing the playback condition each time.

This embodiment is not limited to the foregoing embodiments. A person with ordinary knowledge in the field can add various changes and improvements within the technical scope of the invention. Modifications will be described below.

### (Modification 1)

In the first embodiment, the dynamic image 23 in which the images 22 of the left and right hands make the same movement is used. The right hand and the left hand may make different movements from each other. A dynamic image 23 in which the images 22 of the left and right hands alternately open and close may be used. A dynamic image 23 with which the patient 3 can easily carry out training can be used.

### (Modification 2)

In the first embodiment, one can input the display condition for the dynamic image 23 while viewing the screen displayed in the dialog section 21b. An operation part such as a rotating dial or a sliding knob may be attached to the input/output terminal 7 so as to enable input of the display condition. One can operate the operation part based on the sensation of touching the operation part without seeing it.

### (Modification 3)

In the first embodiment, a planar image is displayed on the head-mounted display 2. A stereoscopic image may be displayed on the head-mounted display 2. It facilitates the patient 3 to perceive the sensation of actually moving the hand 3c.

### (Modification 4)

In the first embodiment, the control device 5 is not connected to an external device. The control device 5 may be connected to a server via the communication device 9 and a network. Then, a plurality of setting data 14 when the patient 3 drives the support device for phantom limb pain treatment 1 is stored in the server. Also, when the patient 3 carries out training, the setting data 14 may be transferred from the server. When a plurality of support device for phantom limb pain treatments 1 is installed, the previous setting data 14 may be utilized even if different devices are used.

### (Modification 5)

In the first embodiment, a pain relief treatment for the hand 3c is carried out using the support device for phantom limb pain treatment 1. A pain relief treatment or rehabilitation of fingers may also be carried out using the support device for phantom limb pain treatment 1. By using the dynamic image 23 in which fingers move, a treatment similar to the first embodiment can be carried out. In this case, too, the patient 3 or the assistant can input a display condition for the dynamic image 23, using the input/output terminal, and the patient 3 can view the dynamic image 23, changing the playback condition each time. In addition, this can be used in various cases such as for elbows and ankles.

### (Modification 6)

In the first embodiment, the mirror part 2a is a non-transmitting mirror. The mirror part 2a may also be a transmitting type. In this case, the dynamic image forming unit 17 forms a dynamic image in such a way that the hand 3c seen through the mirror part 2a and the image 22 of the hand in the dynamic image are superimposed on each other. Thus, the patient 3 can have the sensation of the paralyzed hand 3c moving. Moreover, a cover may be attached to the mirror part 2a so as to switch between the transmitting type and the non-transmitting type. A type which enables the patient to have the sensation of the hand 3c moving more easily is selected.

### (Modification 7)

In the first embodiment, the support device for phantom limb pain treatment 1 is used for the pain relief treatment in which the patient tries to move the missing hand 3c. In addition, this may also be used for rehabilitation of a body part that has become hard to move due to nerve or brain damage. Viewing the dynamic image, the patient can train the nervous system by moving the body part that has become hard to move.

In the first embodiment, the dynamic image 23 in which the image 22 of the hand as a target object moves is displayed. However, the dynamic image to be displayed is not limited to this. It may be another part of the body, and not only the actual body shot in an image but also a simulated body based on an animation or the like may be displayed. Also, as an object to be displayed, not only those belonging to the body but also a totally different object may be displayed. The totally different object is not particularly limited but may be, for example, a book or a door. That is, it may be anything that the patient can visually recognize or understand as making a movement similar to a repetition movement of the body.

### Reference Signs List

1 ... support device for phantom limb pain treatment as a display device, 2 ... head-mounted display as a dynamic image display unit, 3 ... patient, 3c ... hand as a part of the body, 7 ... input/output terminal as a display condition setting unit, 10 ... CPU as a computing unit, 11 ... storage section as a storage unit, 13 ... dynamic image data, 16 ... display condition input unit as a display condition setting unit, 18 ... dynamic image transmitting unit as a dynamic image data output unit, 22 ... image of a hand as a target object, 23 ... dynamic image, 27 ... forward speed input section as a display condition setting unit, 28 ... backward speed input section as a display condition setting unit, 34 ... backward waiting as a backward waiting state, 34a ... backward waiting time, 35 ... forward waiting as a forward waiting state, 35a ... forward waiting time, 36 ... first state, 37 ... second state.

## Claims

1. A display device comprising:
a dynamic image data output unit which outputs dynamic image data in which a target object carries out a repetition movement between a first state and a second state;
a dynamic image display unit which displays a dynamic image based on the dynamic image data; and
a display condition setting unit capable of setting a display condition for the repetition movement,
wherein the display condition includes a forward speed which is a speed of shifting from the first state to the second state and a backward speed which is a speed of shifting from the second state to the first state.

2. The display device according to claim 1, wherein
the dynamic image has a forward shift of shifting from the first state to the second state and a backward shift of shifting from the second state to the first state,
a forward waiting state of waiting for a forward waiting time between the backward shift and the forward shift, and
a backward waiting state of waiting for a backward waiting time between the forward shift and the backward shift, and
the display condition includes the forward waiting time and the backward waiting time.

3. The display device according to claim 1 or 2, wherein
the dynamic image includes images corresponding to a plurality of states of the target object, and the display condition includes an image corresponding to the first state and an image corresponding to the second state.

4. The display device according to one of claims 1 to 3, wherein
the dynamic image display unit is a head-mounted display.

5. A display method comprising:
displaying dynamic image data in which a repetition movement between a first state and a second state is carried out, according to a display condition for a dynamic image that is set,
wherein the display condition includes a forward speed which is a speed of shifting from the first state to the second state and a backward speed which is a speed of shifting from the second state to the first state.

6. A program causing a computer to function as:
a display condition accepting unit which accepts a display condition for a dynamic image; and
a dynamic image data output unit which outputs dynamic image data corresponding to the display condition to a display unit,
wherein the display condition includes a forward speed which is a speed at which a target object shifts from a first state to a second state and a backward speed which is a speed at which the target object shifts from the second state to the first state.
